# EUROPEAN PATENT SPECIFICATION

(11) **EP 3 705 032 B1**
(45) Date of publication and mention of the grant of the patent: **19.08.2026**
(21) Application number: 18872032.0
(22) Date of filing: 02.11.2018
(51) Int. Cl.: A61B 5/021, G06N 99/00, A61B 5/00, G16H 30/20, G16H 50/30

(54) **OPEN API-BASED MEDICAL INFORMATION PROVIDING METHOD AND SYSTEM**
VERFAHREN UND SYSTEM ZUR BEREITSTELLUNG VON MEDIZINISCHEN INFORMATIONEN AUF OFFENER API-BASIS
PROCÉDÉ ET SYSTÈME DE FOURNITURE D'INFORMATIONS MÉDICALES BASÉ SUR UNE API OUVERTE

(30) Priority: 03.11.2017 KR 20170146158
(43) Date of publication of application: 09.09.2020
(73) Proprietor: Deepmedi Inc., Gwangju 61005 (KR)
(72) Inventor: LEE, Kwang Jin, Suwon-si Gyeonggi-do 16697 (KR); CHO, Dong Rea, Suwon-si Gyeonggi-do 16697 (KR); KIM, Jong In, Yongin-si Gyeonggi-do 17066 (KR)
(74) Representative: Morabito, Sara
(86) International application number: PCT/KR2018/013271
(87) International publication number: WO 2019/088769

(56) References cited:
- WO-A1-2017/005016
- WO-A1-2017/073371
- KR-A- 20100 099 881
- KR-B1- 100 963 698
- US-A1- 2011 293 179
- US-A1- 2017 278 546
- ALZAHRANI AHMED: "Preprocessing Realistic Video for Contactless Heart Rate Monitoring Using Video Amplification Methodologies", 1 January 2015 (2015-01-01), XP055823859, Retrieved from the Internet <URL:https://curve.carleton.ca/system/files/etd/299cf146-7120-4c16-9c67-f9eaf8e741c0/etd_pdf/c0f3ee88deced43e1595d7f9cf68b85a/alzahrani-preprocessingrealisticvideoforcontactless.pdf>
- JENSEN JANUS NØRTOFT: "Camera-based Heart Rate Monitoring", 1 January 2014 (2014-01-01), XP055823834, Retrieved from the Internet <URL:http://www2.imm.dtu.dk/pubdb/edoc/imm6796.pdf> [retrieved on 20210713]

## Description

### TECHNICAL FIELD

One or more embodiments relate to a method and system for providing medical information based on an open application programming interface (API).

### BACKGROUND ART

Bio signals include various pieces of information indicating health states. Accordingly, a bio signal of an examination subject is measured, and a current health status of the examination subject can be ascertained from the measured bio signal. One of the bio signals that are widely measured for this purpose is blood pressure.

Accordingly, various studies have been conducted on blood pressure monitors that allow examination subjects to easily measure their blood pressures. In particular, the development of the electronics industry has produced automated blood pressure monitors capable of measuring blood pressure in an indirect manner. For example, WO2017005016 discloses a networked medical monitoring system using an open API to provide blood pressure information based on cardiovascular signals. However, such systems generally rely on dedicated bio-sensors, such as photoelectric or electrode sensors (e.g., PPG or ECG), and do not suggest the use of ubiquitous camera sensors for deriving blood pressure from image signals. Conventional blood pressure monitors are relatively big and thus difficult to carry, and whenever these conventional blood pressure monitors measure blood pressure, a cuff needs to be worn, which is cumbersome.

As various bio signal measurement devices, for example, a photoplethysmogram (PPG) sensor, an electrocardiogram (ECG) sensor, and a camera sensor, have recently become commonplace, they can be mounted on electronic apparatuses including smart watches, medical apparatuses, home IoT devices, smartphones, etc., and can thus be easily carried, so that users may conveniently check their health information.

However, these electronic apparatuses themselves cannot accurately analyze collected bio information and provide medical information based on a result of the analysis.

### DESCRIPTION OF EMBODIMENTS

### TECHNICAL PROBLEM

Provided are a method and system for providing open application programming interface (API)-based medical information.

According to embodiments, examination subjects may easily measure their blood pressures and check their accurate analysis results.

Moreover, health-care services and manufacturers of electronic apparatuses or portable medical devices may easily call an open API without developing a special algorithm and special system for analyzing a measured bio signal and estimating medical information, thereby easily providing processed medical information to users.

Additional aspects will be set forth in part in the description which follows and, in part, will be apparent from the description, or may be learned by practice of the presented embodiments.

### SOLUTION TO PROBLEM

An open application programming interface (API)-based medical information providing method according to an embodiment includes receiving user information and a bio information measurement signal through a network according to a call of the open API protocol in at least one electronic apparatus that is connected through the network and includes a bio information measurement device; performing user authentication, based on the received user information, and estimating at least one medical information from among heart rate information, stress index information, cardiovascular disease dangerousness index information, and blood pressure information, based on the user information and the bio information measurement signal; and transmitting the estimated at least one medical information to the at least one electronic apparatus through the network.

The bio information measurement device is a camera sensor, and the bio information measurement signal is an image signal corresponding to a finger or face photographed by the camera sensor.

The at least one electronic apparatus may be one of a smartwatch, a smartphone, a medical apparatus, or an IoT device.

The at least one electronic apparatus may include an open API client module to call the open API protocol.

The estimating of the at least one medical information includes transforming an RGB image of the image signal into an HSV image, changing a V channel value of the HSV image to a preset value, transforming the HSV image having the preset value as its V channel value into an RGB image, and estimating the blood pressure information by using the RGB image and a previously machine-learned blood pressure estimation model.

The estimating of the at least one medical information includes transforming an RGB image of the image signal into an optical flow image, extracting a motion vector from the optical flow image, extracting a blood flow rate, based on the extracted motion vector, and estimating the blood pressure information by using data about the extracted blood flow rate and a previously machine-learned blood pressure estimation model.

The user information may include a height, a gender, an age, and a weight.

An open API-based medical information providing system is defined in claim 1.

The bio information measurement device is a camera sensor, and the bio information measurement signal is an image signal corresponding to a finger or face photographed by the camera sensor.

The at least one electronic apparatus may be one of a smartwatch, a smartphone, a medical apparatus, or an IoT device.

The at least one electronic apparatus may include an open API client module to call the open API protocol.

The data processing module may be further configured to transform an RGB image of the image signal into an HSV image, change a V channel value of the HSV image to a preset value, transform the HSV image having the preset value as its V channel value into an RGB image, and estimate the blood pressure information by using the RGB image and a previously machine-learned blood pressure estimation model.

The data processing module may be further configured to transform an RGB image of the image signal into an optical flow image, extract a motion vector from the optical flow image, extract a blood flow rate, based on the extracted motion vector, and estimate the blood pressure information by using data about the extracted blood flow rate and a previously machine-learned blood pressure estimation model.

The blood pressure estimation model may be learned via machine learning based on collected pieces of blood pressure information of a plurality of objects and corrected images obtained by correcting collected images of the plurality of objects and the pieces.

A non-transitory computer-readable recording medium according to another embodiment has recorded thereon a program for executing the open API-based medical information providing method.

### ADVANTAGEOUS EFFECTS OF DISCLOSURE

In a method and system for providing open application programming interface (API)-based medical information, examination subjects may easily measure their blood pressures and check their accurate analysis results, and health-care services and manufacturers of electronic apparatuses or portable medical devices may easily call an open API without developing a special algorithm and special system for analyzing a measured bio signal and estimating medical information, thereby easily providing processed medical information to users.

### BRIEF DESCRIPTION OF DRAWINGS

FIG. 1 is a block diagram of a blood pressure estimation model generation system, according to an embodiment of the disclosure;
FIGS. 2A and 2B are conceptual diagrams of a blood pressure estimation model according to an embodiment of the disclosure;
FIG. 3 is a flowchart of a blood pressure estimation model generation method according to another embodiment of the disclosure;
FIG. 4 is a block diagram of a blood pressure estimation system according to another embodiment of the disclosure;
FIG. 5 is a flowchart of a blood pressure estimation method according to another embodiment of the disclosure;
FIG. 6 is an exemplary view for explaining transformation of each frame of an image into an optical flow image as a blood pressure estimation model;
FIGS. 7A and 7B are exemplary views for explaining extraction of motion vectors from an optical flow image;
FIGS. 8A and 8B are exemplary views for explaining estimation of a blood flow rate from optical flow images;
FIG. 9 is a graph for explaining estimation of blood pressure based on a blood flow rate estimated from optical flow images;
FIG. 10A is a schematic diagram illustrating training of a blood pressure estimation model according to another embodiment of the disclosure;
FIG. 10B is a schematic diagram illustrating estimation of blood pressure by using a blood pressure estimation model according to another embodiment of the disclosure;
FIG. 11 is a schematic diagram of an open application programming interface (API)-based medical information providing system according to another embodiment of the disclosure;
FIGS. 12 through 18 illustrate various pieces of medical information that are output by a system, based on various bio information measurement signals and user information according to embodiments;
FIG. 19 is an exemplary view for describing measurement of stress by using a rear camera of a mobile terminal according to an embodiment;
FIG. 20 is a schematic block diagram of the mobile terminal of FIG. 19;
FIG. 21 is a schematic block diagram of a pulse wave signal and stress measuring apparatus according to an embodiment;
FIG. 22 is a schematic block diagram of a pulse wave signal and stress measuring apparatus according to another embodiment; and
FIGS. 23A through 24D are exemplary diagrams for explaining pulse wave signal and stress measuring methods according to other embodiments.

### MODE OF DISCLOSURE

Embodiments of the disclosure are described in detail herein with reference to the accompanying drawings so that this disclosure may be easily performed by one of ordinary skill in the art to which the disclosure pertain. The disclosure may, however, be embodied in many different forms and should not be construed as being limited to the embodiments set forth herein. In the drawings, parts irrelevant to the description are omitted for simplicity of explanation, and like numbers refer to like elements throughout.

Although general terms widely used at present were selected for describing the disclosure in consideration of the functions thereof, these general terms may vary according to intentions of one of ordinary skill in the art, case precedents, the advent of new technologies, and the like. Hence, the terms must be defined based on their meanings and the contents of the entire specification, not by simply stating the terms.

The terms used in the disclosure are merely used to describe particular embodiments, and are not intended to limit the scope of the disclosure as defined by the appended claims. An expression used in the singular encompasses the expression of the plural, unless it has a clearly different meaning in the context. Throughout the specification, when an element is referred to as being "connected" or "coupled" to another element, it can be directly connected or coupled to the other element, or can be electrically connected or coupled to the other element with intervening elements interposed therebetween. In addition, the terms "comprises" and/or "comprising" or "includes" and/or "including" when used in this disclosure, specify the presence of stated elements, but do not preclude the presence or addition of one or more other elements.

The use of the terms "a" and "an" and "the" and similar referents in the context of describing the disclosure (especially in the context of the following claims) are to be construed to cover both the singular and the plural. Also, the steps of all methods described herein can be performed in any suitable order unless otherwise indicated herein or otherwise clearly contradicted by context. Embodiments of the disclosure are not limited to the described order of the operations.

Thus, the expression ""according to some embodiments" or "according to an embodiment" used in the entire disclosure does not necessarily indicate the same embodiment.

The aforementioned embodiments may be described in terms of functional block components and various processing steps. Some or all of such functional blocks may be realized by any number of hardware and/or software components configured to perform the specified functions. For example, functional blocks according to the disclosure may be realized by one or more microprocessors or by circuit components for a predetermined function. In addition, for example, functional blocks according to the disclosure may be implemented with any programming or scripting language. The functional blocks may be implemented in algorithms that are executed on one or more processors. Furthermore, the disclosure described herein could employ any number of conventional techniques for electronics configuration, signal processing and/or control, data processing and the like. The words "mechanism," "element," "means," and "configuration" are used broadly and are not limited to mechanical or physical embodiments,

Furthermore, the connecting lines or connectors between components shown in the various figures presented are intended to represent exemplary functional relationships and/or physical or logical couplings between the components. Connections between components may be represented by many alternative or additional functional relationships, physical connections or logical connections in a practical device.

The disclosure will now be described more fully with reference to the accompanying drawings, in which exemplary embodiments are shown.

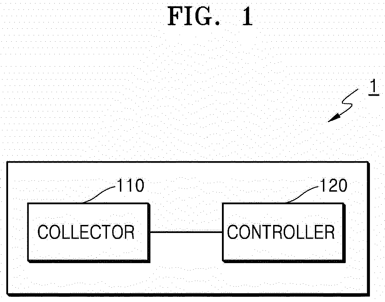

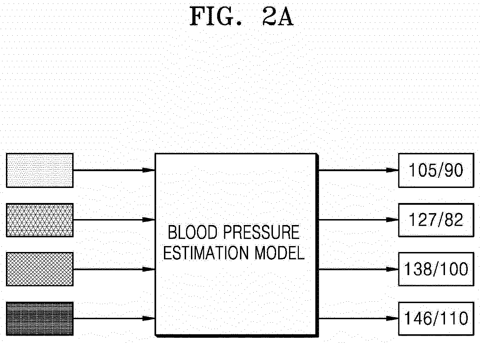

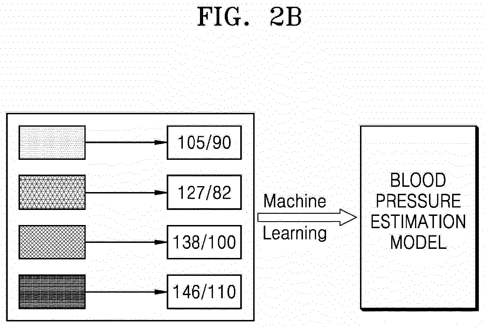

FIG. 1 is a block diagram of a structure of a blood pressure estimation model generation system according to an embodiment of the disclosure, and FIGS. 2A and 2B are conceptual diagrams of a blood pressure estimation model according to an embodiment of the disclosure.

Referring to FIG. 1, a blood pressure estimation model generation system 1 includes a collector 110 and a controller 120.

The collector 110 collects images of a plurality of objects and pieces of blood pressure information of the plurality of objects. The images of the plurality of objects are images obtained by photographing portions of the bodies of a plurality of persons. A portion of a body may be, for example, a portion of the finger of a person, or the face of the person.

A method in which the collector 110 collects the images and the pieces of blood pressure information is not limited to a particular method. For example, the collector 110 may receive and collect the images and the pieces of blood pressure information from an external source. In other words, the images and the pieces of blood pressure information may be input from an external source to the collector 110. Alternatively, the collector 110 may collect the images by photographing the plurality of objects, and may measure and collect the pieces of blood pressure information of the plurality of objects.

The controller 120 corrects the images collected by the collector 110. The controller 120 transforms the collected images from RGB format to HSV format to produce HSV images, and inversely transforms the HSV images of which V values have been changed to 1 back to RGB images. The controller 120 may extract only G-channel images from the RGB images. The controller 120 generates a blood pressure estimation model by training only the extracted G-channel images through machine learning.

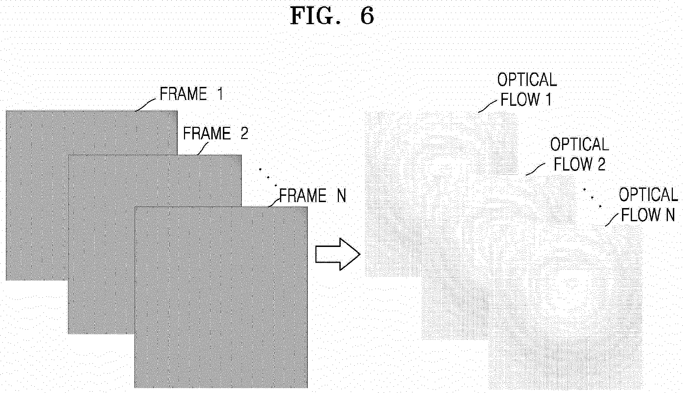

The controller 120 may transform the collected RGB images into optical flow images, extract respective motion vectors from the optical flow images for each frame, estimate a blood flow rate from the motion vectors, and extract pieces of data about the estimated blood flow rate. The controller 120 generates a blood pressure estimation model by training only the extracted pieces of data about the estimated blood flow rate through next machine learning. The generation of the blood pressure estimation model through the optical flow images will be described later with reference to FIGS. 6 through 11.

The controller 120 generates the blood pressure estimation model through machine learning based on the corrected images and the pieces of blood pressure information collected by the collector 110. The machine learning means an algorithm or technology enabling an appropriate operation to be performed on new data based on learned contents by using several pieces of data, and may mean the same thing as a neural network.

The machine learning or the neural network may be a group of algorithms that learn a method of recognizing an object from a certain image input to the neural network, based on an artificial intelligence (AI). For example, the neural network may learn a method of recognizing an object from an image, based on supervised learning using a certain image as an input value and unsupervised learning of discovering a pattern for recognizing an object from an image, by self-learning the type of data necessary for recognizing an object from an image without supervision. For example, the neural network may learn a method of recognizing an object from an image, by using reinforcement learning using a feedback regarding whether a result of recognizing an object according to learning.

The neural network performs an operation for inferring and prediction according to the AI technology. In detail, the neural network may be a deep neural network (DNN) that performs an operation through a plurality of layers. When the number of layers is plural according to the number of internal layers that perform an operation, i.e., when the depth of the neural network performing an operation increases, the neural network may be classified as a DNN. A DNN operation may include a convolution neural network (CNN) operation and the like. In other words, the controller 120 may implement a data recognition model for recognizing an object via the above-illustrated neural network, and may train the implemented data recognition model by using learning data. The controller 120 may analyze or classify an image, which is input data, by using the trained data recognition model, and thus analyze and classify an object included in the image.

Referring to FIG. 2A, the blood pressure estimation model is a model generated from correspondences between respective images of a plurality of objects and pieces of blood pressure information of the plurality of objects, and is used to estimate a blood pressure of an examination subject, based on an image of a portion of the body of the examination subject. In other words, the blood pressure estimation model may estimate a systolic blood pressure and a diastolic blood pressure of the examination subject from an input image of a portion of the body of the examination subject.

The plurality of objects are portions of the bodies of a plurality of persons. The portions of the bodies of the plurality of persons and the portion of the body of the examination subject need to be the same body part, but the examination subject is not necessarily included in the plurality of persons. A portion of a body may be, for example, a tip of a finger.

The controller 120 generates the blood pressure estimation model through machine learning by using the corrected images and the pieces of blood pressure information as learning data.

In detail, referring to FIG. 2B, the controller 120 generates the blood pressure estimation model through machine learning using the corrected images as an input and using the pieces of blood pressure information as a target. In other words, through machine learning using each of the corrected images as an input and using, as a target, blood pressure information of an object from which the corrected image has been obtained, the controller 120 learns a correspondence between each of the corrected image and the blood pressure information and generates the blood pressure estimation model from a result of the learning.

A machine learning algorithm that is used in machine learning is not specified to a particular machine learning algorithm, and may be any machine learning algorithm as long as it is capable of learning the correspondence between each image and blood pressure information and generating a blood pressure estimation model from a result of the learning.

The controller 120 may store the generated blood pressure estimation model therein.

The blood pressure estimation model generation system 1 according to the present embodiment may further include a server (not shown) that stores the generated blood pressure estimation model. The controller 120 may store the generated blood pressure estimation model in an external server. The blood pressure estimation model generation system 1 according to the present embodiment may further include a communication interface (not shown) capable of transmitting the generated blood pressure estimation model to the external server.

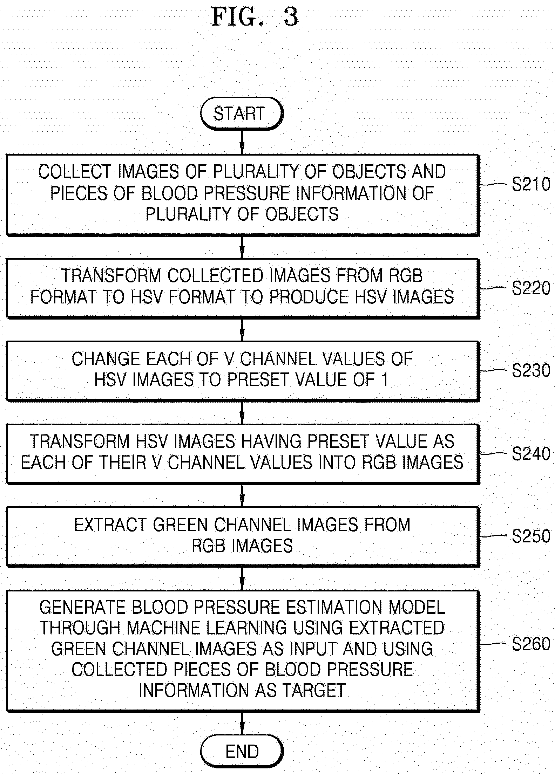

FIG. 3 is a flowchart of a blood pressure estimation model generation method according to another embodiment of the disclosure.

Referring to FIG. 3, the blood pressure estimation model generation method according to the present embodiment includes operation S210 of collecting images of a plurality of objects and pieces of blood pressure information of the plurality of objects, operation S220 of transforming the collected images from RGB format to HSV format to produce HSV images, operation S230 of changing each of the V channel values of the HSV images to a preset value, operation S240 of transforming the HSV images having the preset value as each of their V channel values into RGB images, operation S250 of extracting green channel images from the RGB images, and operation S260 of generating a blood pressure estimation model through machine learning using the extracted green channel images as an input and using the collected pieces of blood pressure information as a target.

The blood pressure estimation model generation method according to the present embodiment is performed by the blood pressure estimation model generation system 1 according to an embodiment of the disclosure.

In operation S210, the images of the plurality of objects and the pieces of blood pressure information of the plurality of objects are collected. The collector 110 collects the images of the plurality of objects and the pieces of blood pressure information of the plurality of objects.

A method in which the collector 110 collects the images and the pieces of blood pressure information is not limited to a particular method. For example, the collector 110 may receive and collect the images and the pieces of blood pressure information from an external source. Alternatively, the collector 110 may collect the images by photographing the plurality of objects, and may measure and collect the pieces of blood pressure information of the plurality of objects.

In operation S220, the collected images are transformed from RGB format to HSV format to produce the HSV images. In operation S230, each of the V channel values of the HSV images is changed to the preset value. In operation S240, the HSV images having the preset value as each of their V channel values are transformed into the RGB images.

Operations S220 through S240 are a process for correcting the images collected by the collector 110, and are thus performed by the controller 120.

The controller 120 transforms the collected images from RGB format to HSV format to produce the HSV images. The controller 120 changes each of the V channel values of the HSV images to the preset value.

A V channel value represents brightness of each image because the images collected by the collector 110 have different brightnesses, the HSV images have different V channel values. The controller 120 changes the different V channel values of the HSV images to the same value. In other words, the controller 120 makes the V channel values of the HSV images identical with each other. For example, the controller 120 changes each of the V channel values of the HSV images to 1.

The controller 120 makes the brightnesses of the HSV images identical with each other and generates the blood pressure estimation model from the brightnesses that are made identical with each other, thereby improving the accuracy of the blood pressure estimation model. The controller 120 transforms the HSV images having the preset value as each of their V channel values into the RGB images.

Operations S220 through S240 are performed for each frame of each image. In other words, the controller 120 performs transformation of an RGB image into an HSV image on each frame of each of the collected images, performs a change of the V channel value of the HSV image to the preset value on each frame of each of the collected images, and performs transformation of the HSV image having the preset value as the V channel value into an RGB image on each frame of each of the collected images.

In operation S250, green channel images are extracted from the RGB images.

Each RGB image includes a red channel image, a green channel image, and a blue channel image. Because a human body absorbs light differently according to the wavelengths of the light, the accuracy of a blood pressure estimation model changes according to from what image from among a red channel image, a green channel image, and a blue channel image the blood pressure estimation model has been generated. In other words, because a blood pressure estimation model generated from a green channel image has highest accuracy, the controller 120 extracts the green channel images from the RGB images and generates the blood pressure estimation model from the extracted green channel images.

In operation S260, the blood pressure estimation model is generated through machine learning using the extracted green channel images as an input and using the collected pieces of blood pressure information as a target.

The controller 120 generates the blood pressure estimation model through machine learning using each of the green channel images as an input and using, as a target, blood pressure information of an object from which the each green channel image has been obtained. The controller 120 may store the generated blood pressure estimation model therein. Alternatively, the controller 120 may store the generated blood pressure estimation model in an external server.

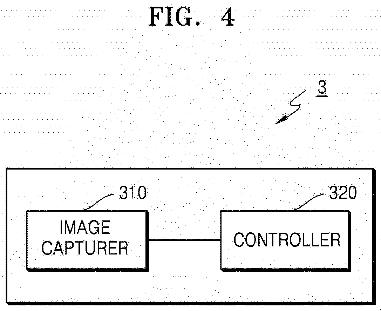

FIG. 4 is a block diagram of a blood pressure estimation system 3 according to another embodiment of the disclosure.

Referring to FIG. 4, the blood pressure estimation system 3 includes an image capturer 310 and a controller 320.

The image capturer 310 captures an image of a portion of the body of an examination subject. The portion of the body of the examination subject is the same as a portion of each of the bodies of a plurality of persons represented in the images collected by the collector 110 according to an embodiment of the disclosure. For example, the portion of the body may be, for example, a tip of a finger of the examination subject. The examination subject does not need to be included in the plurality of persons.

The controller 320 corrects the images captured by the image capturer 310. The controller 320 estimates the blood pressure of the examination subject by using the corrected images and a blood pressure estimation model. The blood pressure estimation model is generated according to the blood pressure estimation model generation method according to another embodiment of the disclosure. The blood pressure estimation model may be stored in the controller 320 or may be stored in an external server. The blood pressure estimation system 3 according to the present embodiment may further include a communication interface (not shown) capable of receiving the blood pressure estimation model from the external server.

The controller 320 may estimate a heart rate of the examination subject from the corrected images. In other words, the controller 320 may estimate the heart rate of the examination subject by using only the corrected images without a special estimation model.

The blood pressure estimation system 3 according to the present embodiment may further include a display (not shown) that outputs the estimated blood pressure and the estimated heart rate of the examination subject so that the examination subject may recognize them. The display outputs the estimated blood pressure and the estimated heart rate of the examination subject by using at least one of a visual method, an auditory method, or a tactile method.

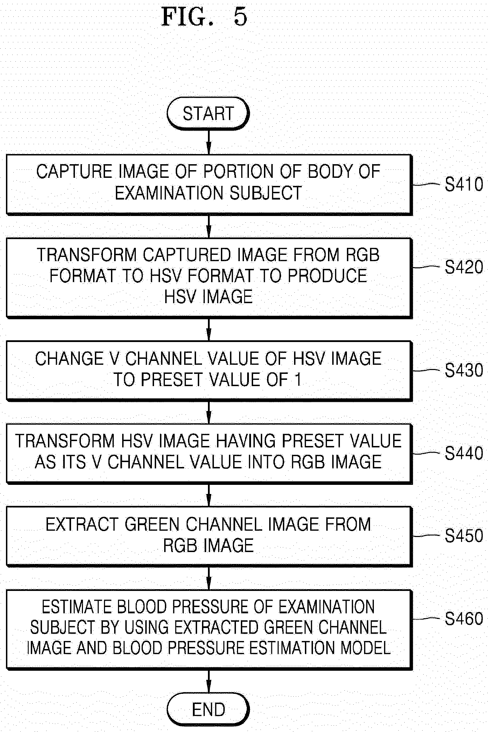

FIG. 5 is a flow chart of a blood pressure estimation method according to another embodiment of the disclosure.

Referring to FIG. 5, the blood pressure estimation method according to the present embodiment includes operation S410 of capturing an image of a portion of the body of an examination subject, operation S420 of transforming the captured image from RGB format to HSV format to produce an HSV image, operation S430 of changing the V channel value of the HSV image to a preset value, operation S440 of transforming the HSV image having the preset value as its V channel value into an RGB image, operation S450 of extracting a green channel image from the RGB image, and operation S460 of estimating the blood pressure of the examination subject by using the extracted green channel image and a blood pressure estimation model.

The blood pressure estimation method according to the present embodiment is performed by the blood pressure estimation system 3 according to another embodiment of the disclosure.

In operation S410, the image of the portion of the body of the examination subject is captured. The image capturer 310 captures the image of the portion of the body of the examination subject. In operation S420, the captured image is transformed from RGB format to HSV format to produce the HSV image. In operation S430, the V channel value of the HSV image is changed to the preset value. In operation S440, the HSV image having the preset value as its V channel value is transformed into the RGB image.

Operations S420 through S440 are a process for correcting the image captured by the image capturer 310, and are thus performed by the controller 320. The controller 320 transforms the captured image from RGB format to HSV format to produce the HSV image. The controller 320 changes the V channel value of the HSV image to the preset value.

As described above, the V channel value represents brightness of each image.

Because the image captured by the image capturer 310 has different brightnesses for different frames, the HSV image has different V channel values for different frames. The controller 320 changes the different V channel values of the HSV images to the same value. In other words, the controller 320 makes the V channel values of all of the frames within the HSV image identical with each other. For example, the controller 320 changes each of the V channel values of all of the frames within the HSV images to 1.

The controller 320 makes the brightnesses of all of the frames within the HSV image identical with each other and estimates the blood pressure of the examination subject from the brightnesses that are made identical with each other, thereby improving the accuracy of blood pressure estimation.

The controller 320 transforms the HSV image having the preset value as its V channel value of each frame into the RGB image.

In operation S450, the green channel image is extracted from the RGB image.

The blood pressure estimation model generated by the blood pressure estimation model generation method according to another embodiment of the disclosure is a blood pressure estimation model generated from the green channel image. Accordingly, the green channel image is used to estimate the blood pressure of the examination subject from the blood pressure estimation model. Thus, the controller 320 extracts the green channel image from the RGB image in operation S450.

In operation S460, the blood pressure of the examination subject is estimated using the extracted green channel image and the blood pressure estimation model.

The controller 320 estimates the blood pressure of the examination subject by using the extracted green channel image and the blood pressure estimation model.

As described above, the blood pressure estimation model generated using the blood pressure estimation model generation method according to another embodiment of the disclosure is the blood pressure estimation model generated from the green channel image, and the blood pressure estimation model has high accuracy compared to a blood pressure estimation model generated from a red channel image or a blue channel image. In other words, in the present operation, because the blood pressure of the examination subject is estimated using the green channel image extracted from the image of the portion of the body of the examination subject and the blood pressure estimation model generated from the green channel image, the blood pressure of the examination subject may be accurately estimated.

FIG. 6 is an exemplary view for explaining transformation of each frame of an image into an optical flow image when the optical flow image is used as the blood pressure estimation model of FIGS. 2A and 2B.

As shown in FIG. 6, frames (frames 1 through N) of each of the images of respective portions of the bodies of a plurality of objects, for example, faces or finger tips, are transformed into optical flow images (optical flows 1 through N). An optical flow is an apparent speed distribution on an image generated by a relative movement between an observer and an object. In general, the optical flow is expressed by an image using an apparent speed vector on each point of an image as a value of a pixel. Moving picture processing may use the following method as a method of calculating the optical flow. When brightness at a time point t in a point (x, y) on an image is I(x, y, t), a difference between temporal and spatial changes of the brightness is approximated as in Equation 1 below.$\frac{dl}{dx} \frac{dx}{dt} + \frac{dl}{dy} \frac{dy}{dt} + \frac{dl}{dt} = 0$
where spatial changes dl/dx and dl/dy and a temporal change dl/dt of the brightness are calculated from a plurality of moving pictures obtained temporally consecutively. Equation 1 gives a restriction on apparent speed vectors dx/dt and dy/dt of an object in the point (x, y). This differential equation alone may not obtain an optical flow uniquely, needs different constraints, and may use, for example, the assumption of a parallel motion and the assumption of the smoothness of a motion.

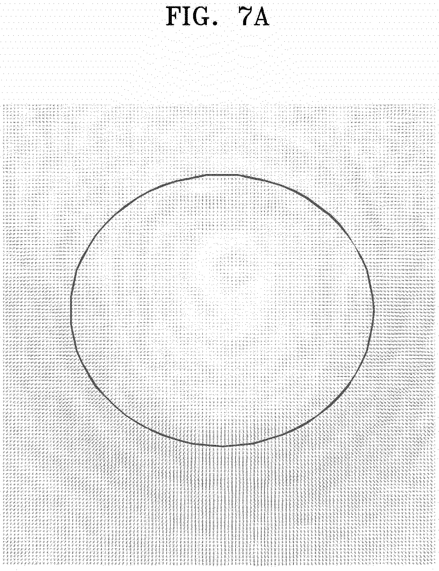

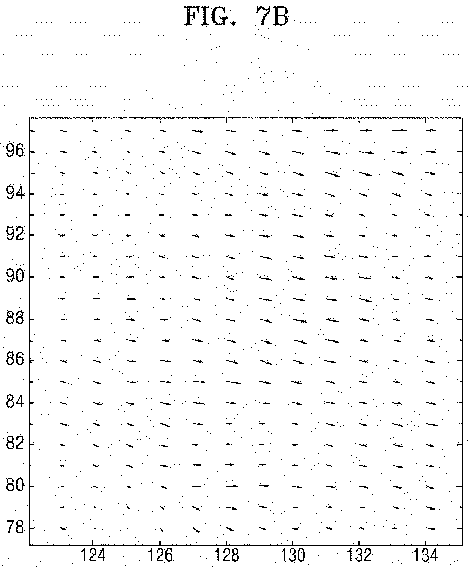

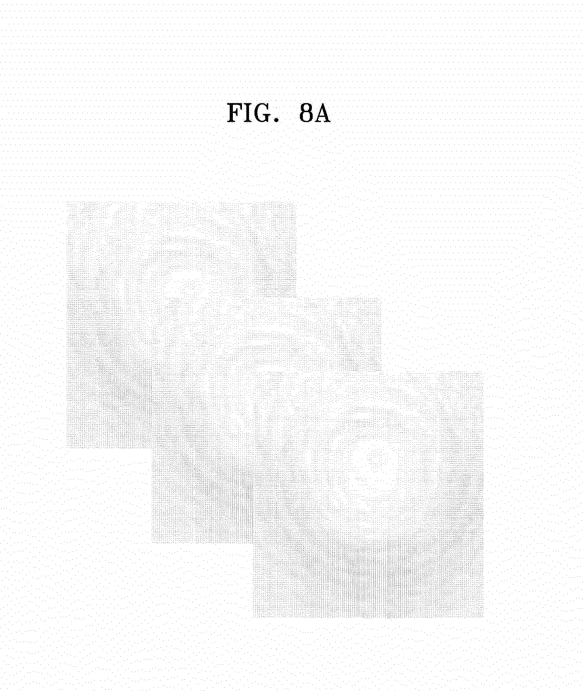

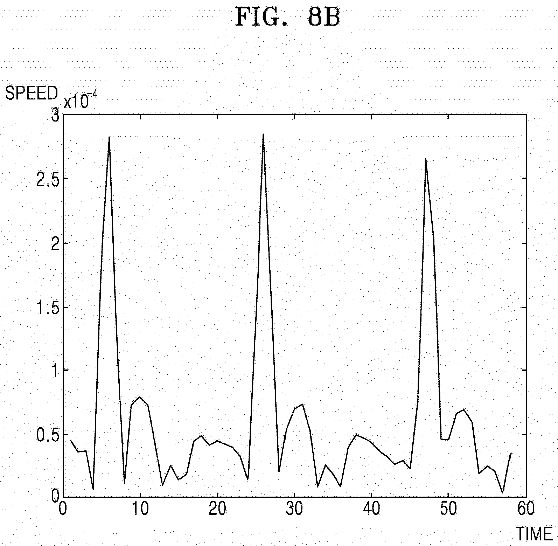

FIGS. 7A and 7B are exemplary views for explaining extraction of motion vectors from an optical flow image. FIGS. 8A and 8B are exemplary views for explaining estimation of a blood flow rate from optical flow images.

A distribution of motion vectors or blood flow rate vectors may be calculated as shown in FIG. 7B, with respect to one region, for example, a center portion, of the optical flow image as shown in FIG. 7A. Then, a distribution of respective blood flow rate vectors for the optical flow images for each frame as shown in FIG. 8A may be represented as a distribution of speed values according to time, as shown in FIG. 8B.

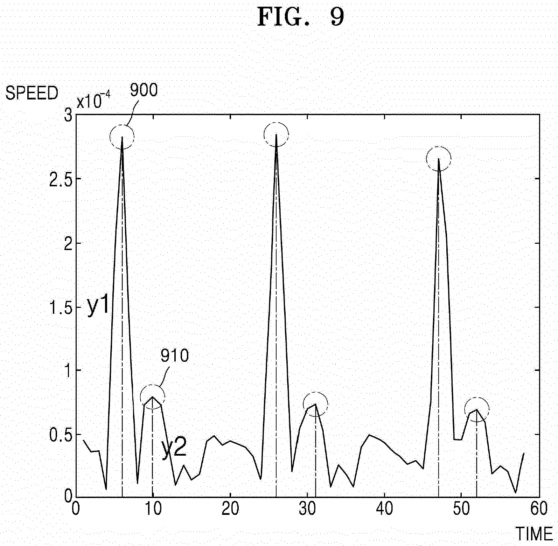

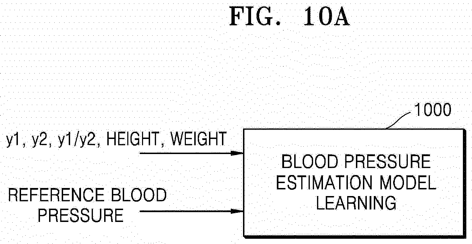

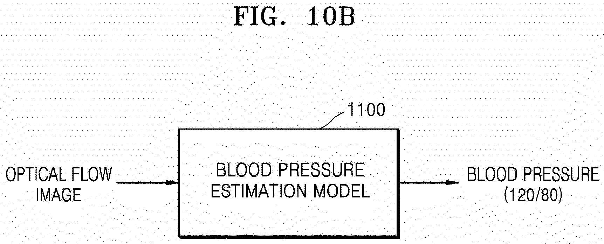

FIG. 9 is a graph for explaining estimation of a blood pressure based on a blood flow rate estimated from optical flow images. FIG. 10A is a schematic diagram illustrating training of a blood pressure estimation model according to another embodiment of the disclosure. FIG. 10B is a schematic diagram illustrating estimation of a blood pressure by using a blood pressure estimation model according to another embodiment of the disclosure.

As shown in FIG. 9, when a distribution of blood flow rates for the optical flow images is calculated, a heartbeat, for example, a feature value 900 of a blood flow rate during contraction of the heart and a feature value 910 of a blood flow rate during relaxation of the heart, may be shown, and each feature value (900 and 910) may be indicated as y1 and y2, respectively. This distribution shows a repetitive pattern (y1 and y2) as shown in FIG. 9.

A blood flow rate may be estimated through the optical flow image described above with reference to FIGS. 6 through 9, and data about this blood flow rate, for example, y1, y2, and y1/y2, and user information including respective heights and weights of objects are matched with a reference blood pressure value, thereby training a blood pressure estimation model 1000 as shown in FIG. 10A.

In this method, the controller 120 of FIG. 1 trains the blood pressure estimation model 1000, based on the images and the pieces of blood pressure information collected by the collector 110. The controller 120 extracts the optical flow images from the corrected images, learns the correspondences between the extracted optical flow images and the pieces of blood pressure information through machine learning, and generates the blood pressure estimation model 1000 from a result of the machine learning.

In detail, the controller 120 transforms the collected RGB images into optical flow images, extracts respective motion vectors from the optical flow images, extracts a blood flow rate, based on each of the extracted motion vectors, and generates the blood pressure estimation model 1000 through machine learning based on data about the extracted blood flow rate, user information, and the pieces of blood pressure information.

Referring to FIG. 10B, the blood pressure estimation system 3 of FIG. 4 estimates a blood pressure from an input optical flow image of an examination subject by using the machine-learned blood pressure estimation model 1000 of FIG. 10A.

The controller 320 of FIG. 4 transforms the captured RGB image into an optical flow image, extracts a motion vector from the optical flow image, extracts a blood flow rate, based on the extracted motion vector, and estimates the blood pressure of the examination subject by using data about the extracted blood flow rate, user information, and the blood pressure estimation model 1000.

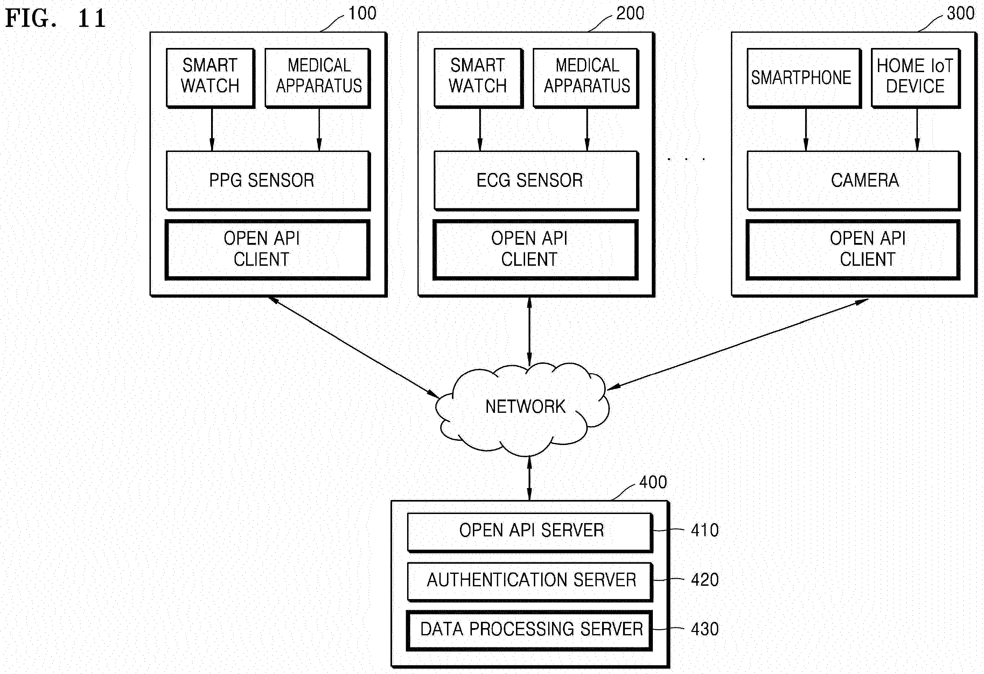

FIG. 11 is a schematic diagram of an open application programming interface (API)-based medical information providing system according to another embodiment of the disclosure.

Referring to FIG. 11, various electronic apparatuses 100 through 300 are connected to an open API-based medical information providing system 400 through a network. The various electronic apparatuses 100 through 300 may be, but are not limited to, a smart watch, a medical apparatus, a smartphone, and a home IoT apparatus. The various electronic apparatuses 100 through 300 include various sensors capable of measuring a bio signal. For example, the various sensors are a camera sensor.

The various electronic apparatuses 100 through 300 of FIG. 11 may be, but are not limited to, wearable apparatuses. These electronic apparatuses 100 through 300 may measure various pieces of bio information The bio information includes image information about a portion of the finger or face photographed by a camera, as described above with reference to FIGS. 1 through 11.

The electronic apparatuses 100 through 300 are able to connect to an open API-based medical information system through a network, and may mount an open API client module thereon to thereby call an open API to request medical information. Accordingly, the electronic apparatuses 100 through 300 may transmit personal information of a user, for example, a height, an age, and a weight, and a PPG signal, an ECG signal, or an image signal from which a pulse wave signal may be estimated, which is measured by a bio signal measuring apparatus, according to an open API protocol.

The open API-based medical information providing system 400 receives user information and a bio information measurement signal from the electronic apparatuses 100 through 300 connected to the open API-based medical information providing system 400 through a network and respectively including bio information measurement apparatuses according to a call of the open API protocol, performs user authentication, estimates blood pressure information based on the user information and the bio information measurement signal, and transmits estimated medical information to the electronic apparatuses 100 through 300 through the network.

The open API-based medical information providing system 400 may include an open API server 410, an authentication server 420, and a data processing server 430.

The open API server 410 communicates with open API clients mounted on the various electronic apparatuses 100 through 300.

The open API server 410 receives the user information and the bio information measurement signal from the open API clients of the electronic apparatuses 100 through 300 according to a call of the open API protocol.

The authentication server 420 performs user authentication, based on the user information received from the electronic apparatuses 100 through 300.

The data processing server 430 estimates blood pressure information based on the user information and the bio information measurement signal, and transmits estimated medical information to the electronic apparatuses 100 through 300 through the network.

The data processing server 430 may implement the blood pressure estimation model generation system and the blood pressure estimation system described above with reference to FIGS. 1 through 11. For example, when the bio information measurement signal is an image signal obtained by a camera sensor, the data processing server 430 may transform an RGB image of the image signal into an HSV image, change the V channel value of the HSV image to a preset value, transform the HSV image having the preset value as its V channel value into an RGB image, and estimate blood pressure information by using the RGB image and a previously machine-learned blood pressure estimation model.

Selectively, the data processing server 430 may transform the RGB image into an optical flow image, extract a motion vector from the optical flow image, extract a blood flow rate, based on the extracted motion vector, and estimate blood pressure information by using data about the extracted blood flow rate and the previously machine-learned blood pressure estimation model.

The data processing server 430 stores the above-described blood pressure estimation model, and a method of generating the blood pressure estimation model and a method of training the blood pressure estimation model are the same as those described above with reference to FIGS. 1 through 11.

An open API-based medical information providing system according to an embodiment may provide, for example, an API for registering user's personal information, an API for extracting a PPG signal from an image, an API for extracting a heart rate from a PPG, an API for extracting a stress index from the PPG, an API for extracting a cardiovascular disease dangerousness index from the PPG, an API for estimating a blood pressure from the PPG, and an API for providing extracted and estimated health indexes according to date.

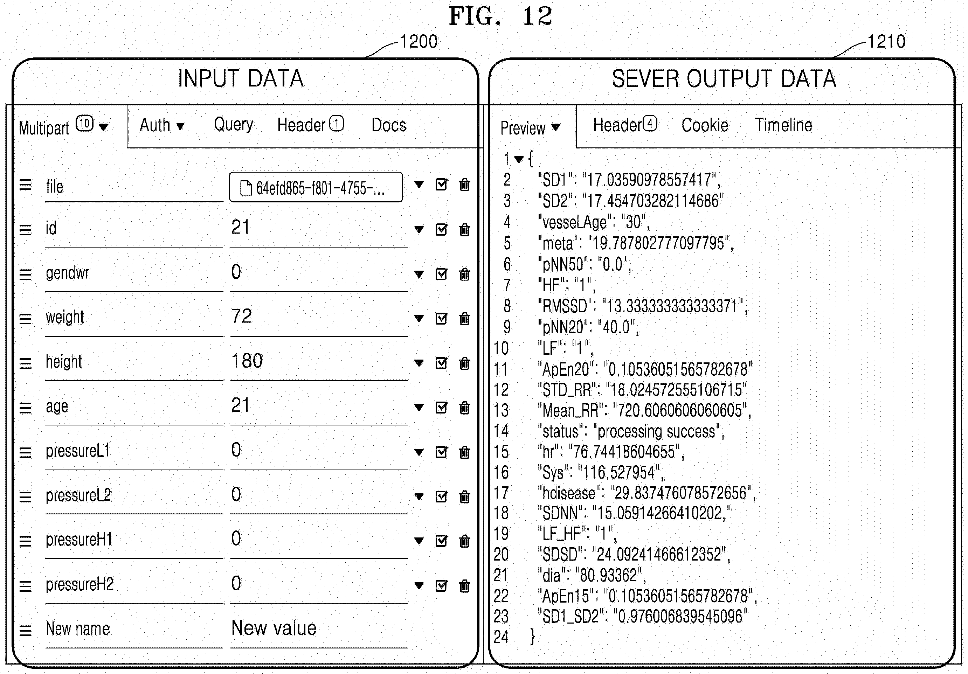

FIGS. 12 through 18 illustrate various pieces of medical information that are output by a system, based on various bio information measurement signals and user information according to embodiments.

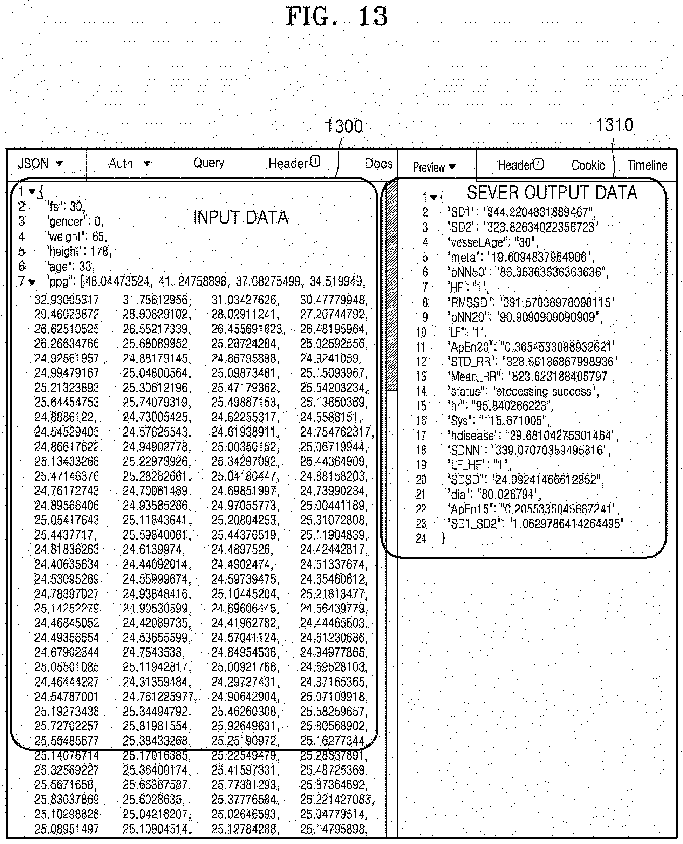

FIGs. 12 and 13 are exemplary APIs for calculating a blood pressure and other health information as information that is transmitted by the system, based on an image signal and a user information input.

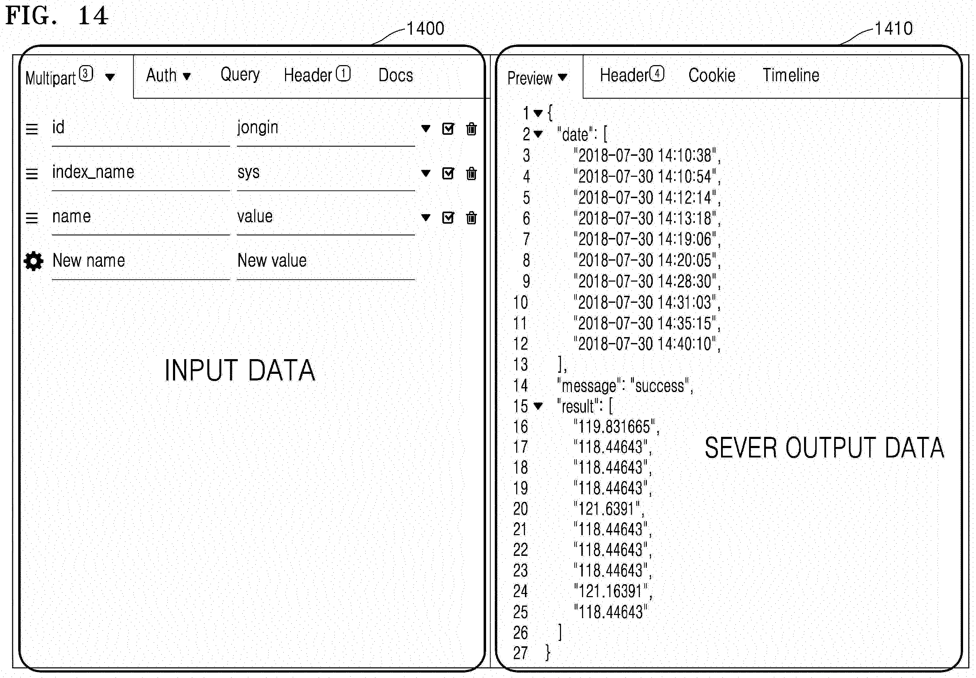

FIG. 14 is an exemplary API for calculating a blood pressure and other health information as information that is transmitted by the system, based on a PPG signal and a user information input.

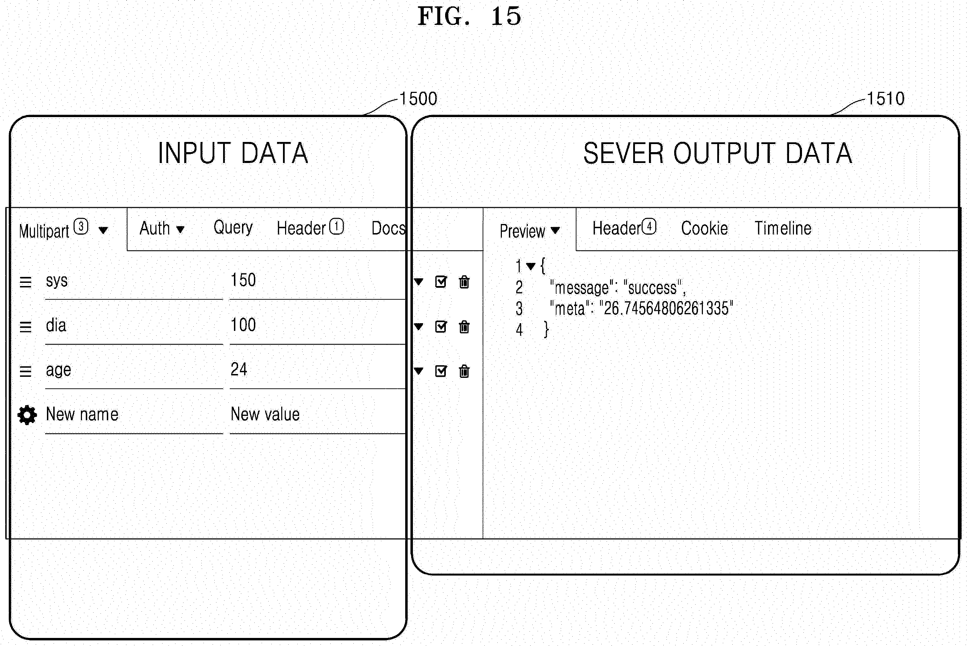

FIG. 15 is an exemplary API for calling a health information history through a user ID input.

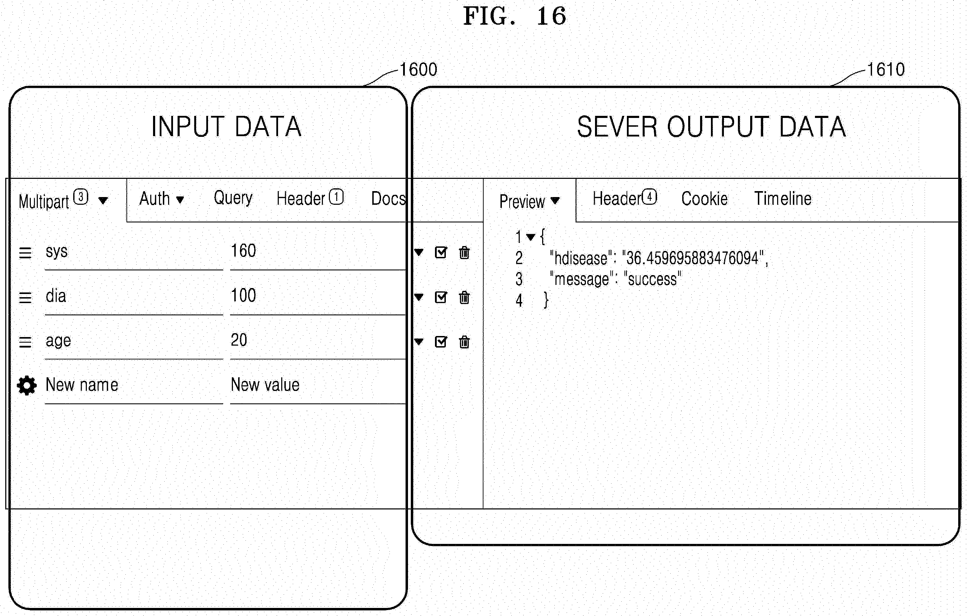

FIG. 16 is an exemplary API for calculating a metabolic syndrome risk through blood pressure and age inputs.

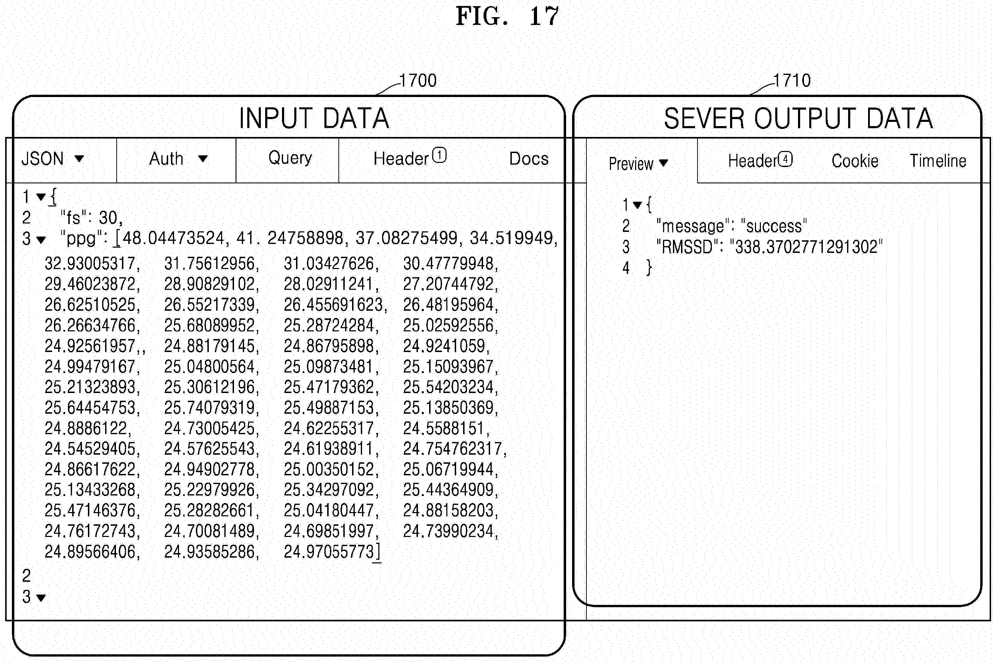

FIG. 17 is an exemplary API for calculating a cardiovascular disease risk through blood pressure and age inputs.

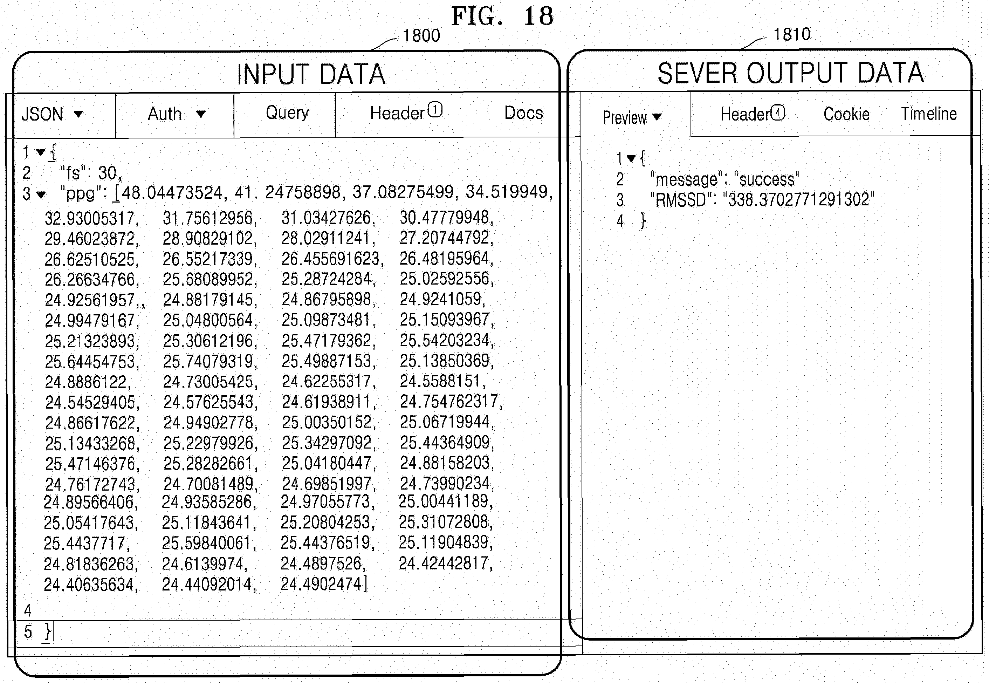

FIG. 18 is an exemplary API for calculating a resistance to stress through a PPG signal and a sampling frequency input.

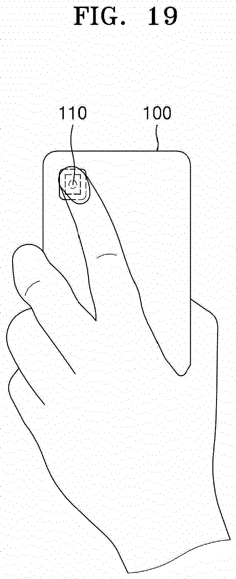

FIG. 19 is an exemplary view for describing measurement of a stress by using a rear camera of a mobile terminal according to an embodiment.

Referring to FIG. 19, a finger of an examination subject is put on a rear camera 110 of a mobile terminal 100 to measure a pulse wave signal. The mobile terminal 100 obtains an image of the finer through a smartphone rear camera and a built-in flash that are generally embedded in the mobile terminal 100. The mobile terminal 100 extracts the pulse wave signal from the obtained image by using several signal processing techniques, for example, by using an infinite impulse response (IIR) filter. The mobile terminal 100 calculates an HRV-related signal by using the extracted pulse wave signal and calculates a stress index by using an HRV-related signal.

The HRV means a minute change difference between a heart beat cycle and a next heart beat cycle. The minute change difference is more used to actually find a clinical meaning than to measure a pulse. This is determined due to an influence of an autonomic nervous system and is related to an interaction between a sympathetic nerve and a parasympathetic nerve. This interaction causes a heart rate to change according to a change in the body inside or an external environment. The heart rate of a healthy person changes greatly and complexly, whereas the heart rate of a person having a disease or being in a stress state significantly decreases in terms of complexity.

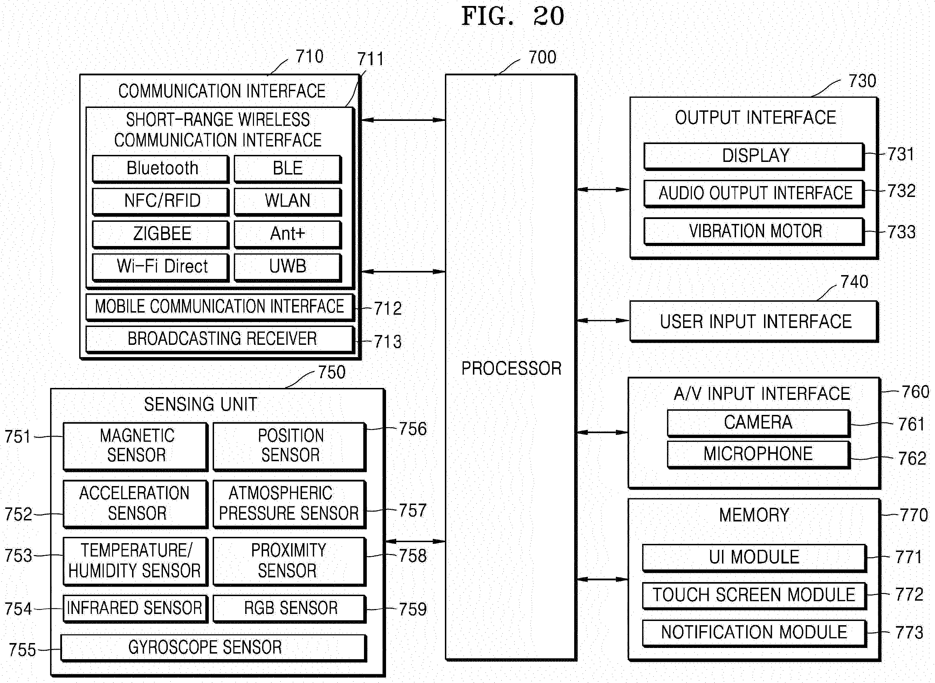

FIG. 20 is a schematic block diagram of the mobile terminal 100 of FIG. 19.

Referring to FIG. 20, the mobile terminal 100 may include a processor 700, a communication interface 710, and an output interface 730. However, all of the illustrated components are not essential. The mobile terminal 100 may be implemented by more or less components than those illustrated in FIG. 20.

For example, the mobile terminal 100 according to an embodiment may further include a user input interface 740, a sensing unit 750, an audio/video (A/V) input interface 760, and a memory 770, in addition to the processor 700, the communication interface 710, and the output interface 730.

The aforementioned components will now be described in detail.

The processor 700 typically controls all operations of the mobile terminal 100. For example, the processor 700 may control the communication interface 710, the output interface 730, the user input unit 740, the sensing unit 750, the A/V input interface 760, and the memory 770 by executing programs stored in the memory 770.

While obtaining a plurality of images of an examination subject through a camera sensor and a built-in flash, the processor 700 may record time information in each of the obtained plurality of images, extract pulse wave signals from the plurality of images, correct the extracted pulse wave signals by using the recorded time information, calculate an HRV from the corrected pulse wave signals, and estimate a stress index by using the calculated HRV.

According to an embodiment, the processor 700 of the mobile terminal 100 measures a stress index. However, the disclosure is not limited thereto, and a mobile terminal may obtain only an image of a finger and transmit image data to an external server (not shown) to calculate a stress index.

The communication interface 710 may include at least one component that enables communication between the mobile terminal 100 and a server (not shown). For example, the communication interface 710 may include a short-range wireless communication interface 711, a mobile communication interface 712, and a broadcasting receiver 713.

Examples of the short-range wireless communication interface 711 may include, but are not limited to, a Bluetooth communication interface, a Bluetooth Low Energy (BLE) communication interface, a near field communication (NFC) interface, a wireless local area network (WLAN) (e.g., Wi-Fi) communication interface, a ZigBee communication interface, an infrared Data Association (IrDA) communication interface, a Wi-Fi direct (WFD) communication interface, an ultra wideband (UWB) communication interface, and an Ant+ communication interface. The mobile communication interface 712 may exchange a wireless signal with at least one selected from a base station, an external terminal, and a server on a mobile communication network. Here, examples of the wireless signal may include a voice call signal, a video call signal, and various types of data according to text/multimedia messages transmission. The broadcasting receiver 713 receives a broadcasting signal and/or broadcasting-related information from an external source via a broadcasting channel. The broadcasting channel may be a satellite channel, a ground wave channel, or the like. According to embodiments, the mobile terminal 100 may not include the broadcasting receiver 713.

The output interface 730 displays information related with the stress index.

The output interface 730 outputs an audio signal, a video signal, or a vibration signal, and may include a display 731, an audio output interface 732, and a vibration motor 733. The display 731 may include, but is not limited to, a key pad, a dome switch, a touch pad (e.g., a capacitive overlay type, a resistive overlay type, an infrared beam type, an integral strain gauge type, a surface acoustic wave type, a piezoelectric type, or the like), a jog wheel, or a jog switch.

The display 731 may be a touch screen in which a touch pad forms a layer structure. The display 731 may include at least one of a liquid crystal display (LCD), a thin film transistor-liquid crystal display (TFT-LCD), an organic light-emitting diode (OLED), a flexible display, a three-dimensional (3D) display, and an electrophoretic display. According to embodiments of a mobile terminal, the mobile terminal 100 may include at least two displays 731.

The audio output interface 732 outputs audio data that is received from the communication interface 720 or stored in the memory 770. The audio output interface 731 also outputs an audio signal (for example, a call signal receiving sound, a message receiving sound, a notification sound) related with a function of the mobile terminal 100. The audio output interface 732 may include, for example, a speaker and a buzzer.

The vibration motor 733 may output a vibration signal. For example, the vibration motor 733 may output a vibration signal corresponding to an output of audio data or video data (for example, a call signal receiving sound or a message receiving sound). The vibration motor 733 may also output a vibration signal when a touch screen is touched.

The user input interface 740 denotes means via which the user inputs data for controlling the mobile terminal 100. For example, the user input interface 740 may be, but is not limited to, a key pad, a dome switch, a touch pad (e.g., a capacitive overlay type, a resistive overlay type, an infrared beam type, an integral strain gauge type, a surface acoustic wave type, a piezo electric type, or the like), a jog wheel, or a jog switch.

The sensing unit 750 may sense the status of the mobile terminal 100 or the status of the surrounding of the mobile terminal 100 and may transmit information corresponding to the sensed status to the processor 700.

The sensing unit 750 may include, but is not limited thereto, at least one selected from a magnetic sensor 751, an acceleration sensor 752, a temperature/humidity sensor 753, an infrared sensor 754, a gyroscope sensor 755, a position sensor (e.g., a global positioning system (GPS)) 756, a pressure sensor 757, a proximity sensor 758, and an RGB sensor 759 (i.e., an illumination sensor). Functions of most of the sensors would be instinctively understood by one of ordinary skill in the art in view of their names and thus detailed descriptions thereof will be omitted herein.

The A/V input interface 760 inputs an audio signal or a video signal, and may include a camera 761 and a microphone 762. The camera 761 may acquire an image frame, such as a still image or a moving picture, via an image sensor in a video call mode or a photography mode. An image captured via the image sensor may be processed by the processor 700 or a separate image processor (not shown).

The image frame obtained by the camera 761 may be stored in the memory 770 or transmitted to the outside via the communication interface 720. At least two cameras 761 may be included according to embodiments of the structure of a mobile terminal. The camera 761 may be provided on the rear surface, as shown in FIG. 19.

The microphone 762 receives an external audio signal and converts the external audio signal into electrical audio data. For example, the microphone 762 may receive an audio signal from an external device or a speaking person. The microphone 762 may use various noise removal algorithms in order to remove noise that is generated while receiving the external audio signal.

The memory 770 may store a program for processing and control by the processor 700, or may store input/output data.

The memory 770 may include at least one type of storage medium selected from among a flash memory type, a hard disk type, a multimedia card micro type, a card type memory (for example, a secure digital (SD) or extreme digital (XD) memory), a random access memory (RAM), a static random access memory (SRAM), a read-only memory (ROM), an electrically erasable programmable ROM (EEPROM), a programmable ROM (PROM), magnetic memory, a magnetic disk, and an optical disk. The mobile terminal 100 may operate a web storage or a cloud server on the internet which performs a storage function of the memory 770.

The programs stored in the memory 770 may be classified into a plurality of modules according to their functions, for example, a user interface (UI) module 771, a touch screen module 772, and a notification module 773.

The UI module 771 may provide a specialized UI, GUI, or the like that interoperates with a mobile terminal, according to application. The touch screen module 772 may detect a touch gesture on a touch screen of a user and transmit information regarding the touch gesture to the processor 700. The touch screen module 772 according to an embodiment of the disclosure may recognize and analyze a touch code. The touch screen module 772 may be configured by separate hardware including a controller.

In order to detect an actual touch or a proximate touch on a touch screen, the touch screen may internally or externally have various sensors. An example of a sensor used to detect a touch on the touch screen is a tactile sensor. The tactile sensor denotes a sensor that detects a touch by a specific object to a degree to which a human feels or more. The tactile sensor may detect various types of information, such as the roughness of a touched surface, the hardness of the touching object, and the temperature of a touched point.

Another example of a sensor used to detect the real touch or the proximity touch on the touch screen is a proximity sensor.

The proximity sensor senses the existence of an object that approaches the predetermined sensing surface or an object that exists nearby, without mechanical contact, by using an electromagnetic force or infrared rays. Examples of the proximity sensor include a transmission-type photoelectric sensor, a direct reflection-type photoelectric sensor, a mirror reflection-type photoelectric sensor, a high frequency oscillation-type proximity sensor, a capacity-type proximity sensor, a magnetic proximity sensor, and an infrared-type proximity sensor. Examples of the touch gesture of the user may include tap, touch and hold, double tap, drag, panning, flick, drag and drop, swipe, and the like.

The notification module 773 may generate a signal for notifying that an event has been generated in the mobile terminal 100. Examples of the event generated in the mobile terminal 100 may include call signal receiving, message receiving, a key signal input, schedule notification, and the like. Examples of the event generated in the mobile terminal 100 may also include generation of a signal informing that a user input has been received, based on a haptic signal that is generated based on a user input received by the display 731.

The notification module 773 may output a notification signal in the form of a video signal via the display 731, in the form of an audio signal via the audio output interface 732, or in the form of a vibration signal via the vibration motor 733.

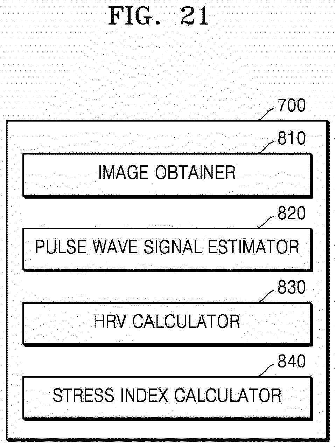

FIG. 21 is a schematic block diagram of a pulse wave signal and stress measuring apparatus 800 according to an embodiment. The pulse wave signal and stress measuring apparatus 800 may be the mobile terminal 100 of FIG. 19, a controller (or a processor) of the mobile terminal 100, or a special measuring apparatus.

Referring to FIG. 21, the pulse wave signal and stress measuring apparatus 800 includes an image obtainer 810, a pulse wave signal estimator 820, an HRV calculator 830, and a stress index calculator 840.

The image obtainer 810 obtains a finger image by photographing a finger. Although a finger is described as an example, the disclosure is not limited thereto, any body part of an examination subject may be photographed. The image obtainer 810 may obtain an image that may be moving picture data of about 30 seconds, and including about 900 frames when at 30 frames per second (fps). Although the 900 frames are used to detect a pulse wave signal, the disclosure is not limited to 900.

The pulse wave signal estimator 820 extracts a pulse wave signal from the obtained image by using several signal processing techniques, for example, by using an IIR filter. The pulse wave signal means a graph as which a heartbeat phenomenon of a peripheral vascular system occurring through contraction and relaxation of the heart is expressed. A technique of detecting a pulse wave cycle on the graph is possible in the time domain and the frequency domain. Examples of the technique possible in the time domain may include peak picking, an auto-correlation function, and an average magnitude difference function (AMDF), and examples of the technique possible in the frequency domain may include RF peak detection and spectrum similarity analysis.

The HRV calculator 830 calculates an HRV-related signal by using the extracted pulse wave signal. The stress index calculator 840 calculates a stress index by using the HRV-related signal. The HRV-related signal calculation and the stress index calculation may use a commonly-known technique.

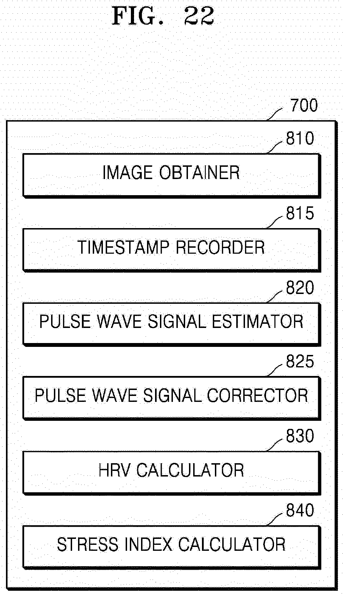

FIG. 22 is a schematic block diagram of a pulse wave signal and stress measuring apparatus 700 according to another embodiment. A description of FIG. 22 that is the same as given above with reference to FIG. 21 will not be repeated herein, and only a difference between FIGS. 21 and 22 will now be described.

Referring to FIG. 22, the pulse wave signal and stress measuring apparatus 900 includes the image obtainer 810, a timestamp recorder 815, the pulse wave signal estimator 820, a pulse wave signal corrector 825, the HRV calculator 830, and the stress index calculator 840.

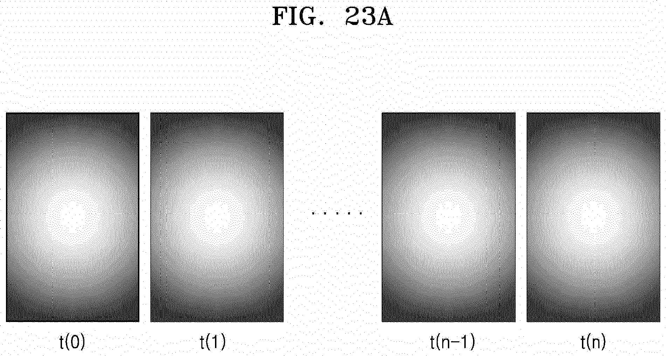

The timestamp recorder 815 separately records time information of each frame obtained by the image obtainer 810, as shown in FIG. 23A. The time information may be, for example, units of milliseconds (ms). Accordingly, it may not be uniform for mobile terminals to obtain frames according to their performance, and thus the timestamp recorder 815 records time information for each frame during image obtainment for stress measurement.

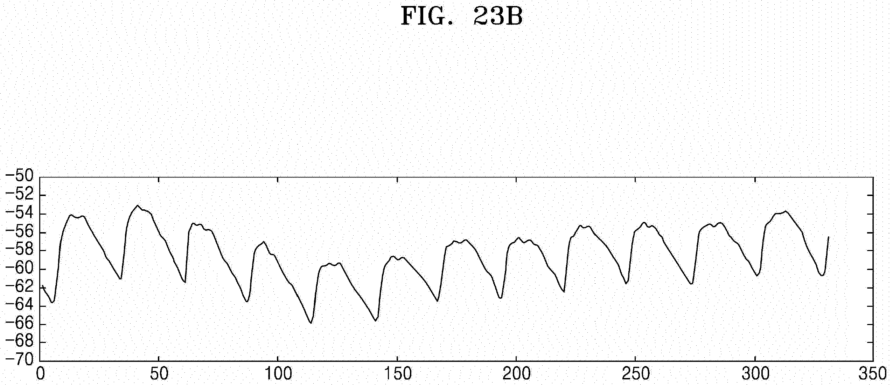

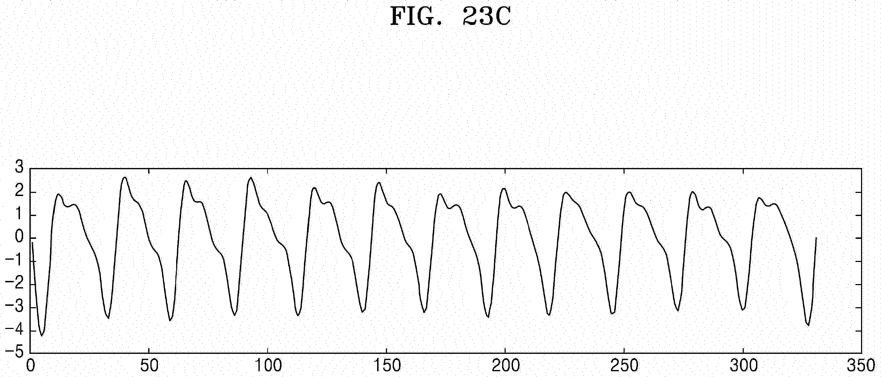

The pulse wave signal estimator 820 averages the G value of the R, G, and B values of the each obtained frame, as shown in FIG. 23B. As shown in FIG. 23C, the pulse wave signal estimator 820 extracts a pulse wave signal through an IIR filter.

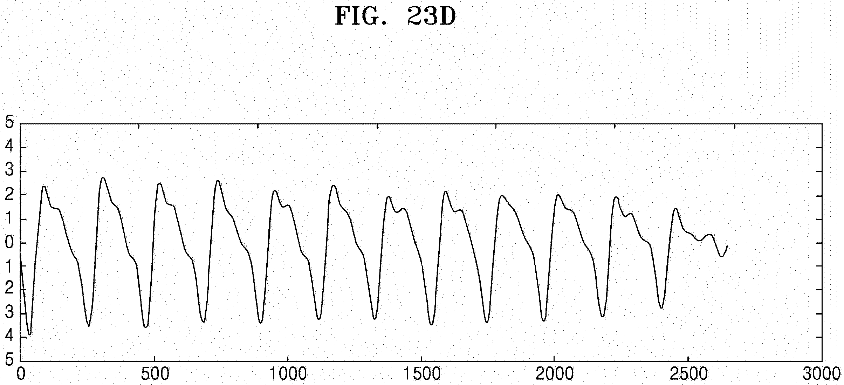

As shown in FIG. 23D, the pulse wave signal corrector 825 corrects the pulse wave signal by applying a cubic spline interpolation method, based on the time information of each frame recorded in the timestamp recorder 815. Although the pulse wave signal is corrected using a cubic spline interpolation method, the disclosure is not limited thereto. Various interpolation methods may be used to correct the pulse wave signal.

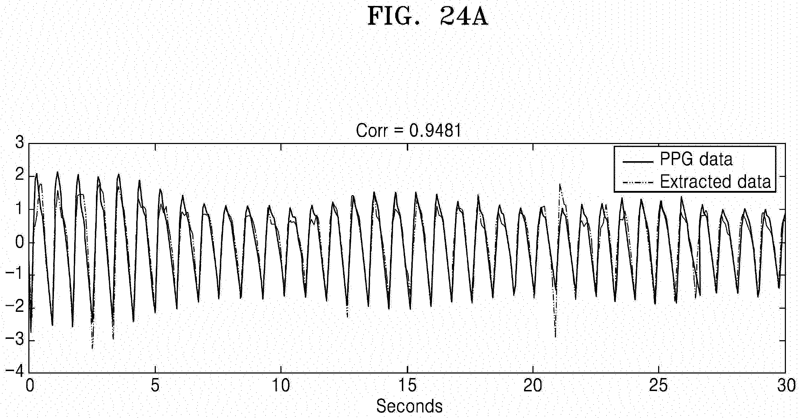

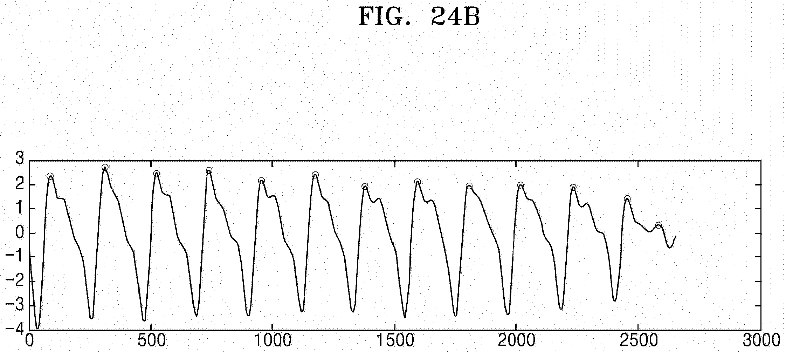

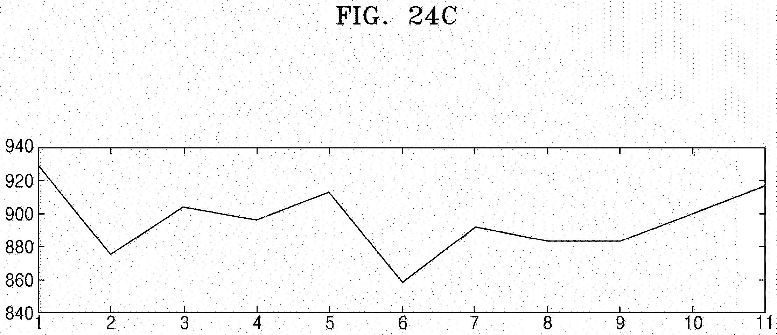

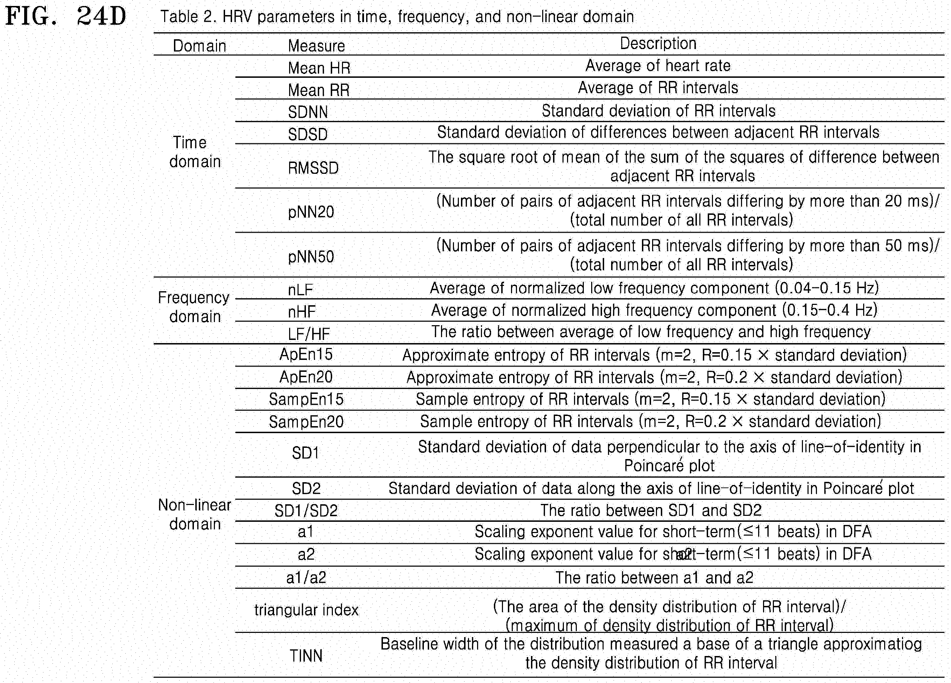

FIGS. 24A through 24D are exemplary diagrams for explaining a pulse wave signal and stress measuring method according to another embodiment.

Referring to FIGS. 24A through 24D, a pulse wave signal for calculating an HRV is extracted from PPG data, and the HRV is calculated using the extracted pulse wave signal. As shown in FIGS. 24A through 24D, a stress index is calculated from the HRV.

Methods and systems for providing open API-based medical information, according to some embodiments, can be embodied as a storage medium including instruction codes executable by a computer such as a program module executed by the computer. A computer readable medium can be any available medium which can be accessed by the computer and includes all volatile/non-volatile and removable/non-removable media. Further, the computer readable medium may include all computer storage and communication media. The computer storage medium includes all volatile/non-volatile and removable/non-removable media embodied by a certain method or technology for storing information such as computer readable instruction code, a data structure, a program module or other data. The communication medium typically includes the computer readable instruction code, the data structure, the program module, or other data of a modulated data signal, or other transmission mechanism, and includes any information transmission medium.

The terminology "~or(er)" or ~ unit" used herein may be a hardware component such as a processor or a circuit, and/or a software component that is executed by a hardware component such as a processor.

A blood pressure estimation model generation system, a blood pressure estimation model generation method, a blood pressure estimation system, and a blood pressure estimation method according to embodiments of the disclosure may be implemented as computer program products including recording media having programs stored therein.

## Claims

1. An open application programming interface (API)-based medical information providing method comprising:
receiving user information and a bio information measurement signal through a network according to a call of the open API protocol in at least one electronic apparatus that is connected through the network and includes a bio information measurement device;
performing user authentication, based on the received user information, and estimating blood pressure information, based on the user information and the bio information measurement signal; and
transmitting the estimated blood pressure information to the at least one electronic apparatus through the network, wherein the bio information measurement device is a camera sensor, and the bio information measurement signal is an image signal corresponding to a finger or face photographed by the camera sensor,
wherein the estimating of the blood pressure information comprises:
transforming a first RGB image of the image signal into an HSV image,
changing a V channel value of the HSV image to a preset value,
transforming the HSV image having the preset value as its V channel value into a second RGB image, extracting a G channel image from the second RGB image, and
estimating the blood pressure information by using the G channel image and a previously machine-learned blood pressure estimation model.

2. The open API-based medical information providing method of claim 1, wherein the at least one electronic apparatus is one of a smartwatch, a smartphone, a medical apparatus, and an IoT device.

3. The open API-based medical information providing method of claim 2, wherein the at least one electronic apparatus includes an open API client module to call the open API protocol.

4. The open API-based medical information providing method of claim 1, wherein the user information comprises a height, a gender, an age, and a weight.

5. The open API-based medical information providing method according to any one of the preceding claims, wherein the blood pressure estimation model is generated from the green channel image.

6. A non-transitory computer-readable recording medium having recorded thereon a program for executing the open API-based medical information providing method of any one of claims 1 through 5.

7. An open application programming interface (API)-based medical information providing system comprising:
an open API module configured to receive user information and a bio information measurement signal through a network according to a call of the open API protocol in at least one electronic apparatus that is connected through the network and includes a bio information measurement device;
an authentication module configured to perform user authentication, based on the received user information; and
a data processing module configured to estimate blood pressure information, based on the received user information and the received bio information measurement signal, and transmit the estimated blood pressure information to the at least one electronic apparatus through the network.,
wherein the bio information measurement device is a camera sensor, and
the bio information measurement signal is an image signal corresponding to a finger or face photographed by the camera sensor.
wherein the data processing module is further configured to
transform a first RGB image of the image signal into an HSV image,
change a V channel value of the HSV image to a preset value,
transform the HSV image having the preset value as its V channel value into a second RGB image, extract a G channel image from the second RGB image, and
estimate the blood pressure information by using the G channel image and a previously machine-learned blood pressure estimation model.

8. The open API-based medical information providing system of claim 7, wherein the at least one electronic apparatus is one of a smartwatch, a smartphone, a medical apparatus, and an IoT device.

9. The open API-based medical information providing system of claim 8, wherein the at least one electronic apparatus includes an open API client module to call the open API protocol.

10. The open API-based medical information providing system according to any one of claims 7-9, wherein the blood pressure estimation model is generated from the green channel image.

## Patentansprüche

1. Verfahren zur Bereitstellung medizinischer Informationen auf Basis einer offenen Anwendungsprogrammierschnittstelle (API), umfassend:
- Empfangen von Benutzerinformationen und eines Bioinformationsmesssignals über ein Netzwerk nach einem Aufruf des offenen API-Protokolls in zumindest einem elektronischen Gerät, das über das Netzwerk verbunden ist und eine Bioinformationsmessvorrichtung umfasst;
- Durchführen einer Benutzerauthentifizierung auf der Basis der empfangenen Benutzerinformationen und Schätzen von Blutdruckinformationen auf der Basis der Benutzerinformationen und des Bioinformationsmesssignals; und
- Übertragen der geschätzten Blutdruckinformationen an zumindest ein elektronisches Gerät über das Netzwerk, wobei die Bioinformationsmessvorrichtung ein Kamerasensor ist und das Bioinformationsmesssignal ein Bildsignal ist, das einem vom Kamerasensor aufgenommenen Finger oder Gesicht entspricht,
- wobei die Schätzung der Blutdruckinformationen aufweist:
- Umwandeln eines ersten RGB-Bildes des Bildsignals in ein HSV-Bild,
- Ändern eines V-Kanal-Werts des HSV-Bildes auf einen voreingestellten Wert,
- Umwandeln des HSV-Bildes, das den voreingestellten Wert als V-Kanal-Wert aufweist, in ein zweites RGB-Bild,
- Extrahieren eines G-Kanal-Bildes aus dem zweiten RGB-Bild, und
- Schätzen der Blutdruckinformationen unter Verwendung des G-Kanal-Bildes und eines zuvor maschinell gelernten Blutdruck-Schätzmodells.

2. Verfahren zur Bereitstellung medizinischer Informationen auf Basis einer offenen API nach Anspruch 1, wobei zumindest ein elektronisches Gerät eine Smartwatch, ein Smartphone, ein medizinisches Gerät oder eine IoT-Vorrichtung ist.

3. Verfahren zur Bereitstellung medizinischer Informationen auf Basis einer offenen API nach Anspruch 2, wobei zumindest ein elektronisches Gerät ein offenes API-Client-Modul umfasst, um das offene API-Protokoll aufzurufen.

4. Verfahren zur Bereitstellung medizinischer Informationen auf Basis einer offenen API nach Anspruch 1, wobei die Benutzerinformationen eine Körpergröße, ein Geschlecht, ein Alter und ein Gewicht aufweisen.

5. Verfahren zur Bereitstellung medizinischer Informationen auf Basis einer offenen API nach einem der vorhergehenden Ansprüche, wobei das Blutdruck-Schätzmodell aus dem Bild des Grünkanals generiert wird.

6. Nichtflüchtiges, computerlesbares Aufzeichnungsmedium, auf dem ein Programm zum Ausführen des Verfahrens zur Bereitstellung medizinischer Informationen auf Basis einer offenen API nach einem der Ansprüche 1 bis 5 aufgezeichnet ist.

7. System zur Bereitstellung medizinischer Informationen auf Basis einer offenen Anwendungsprogrammierschnittstelle (API), umfassend:
- ein offenes API-Modul, das eingerichtet ist, um Benutzerinformationen und ein Bioinformationsmesssignal über ein Netzwerk nach einem Aufruf des offenen API-Protokolls in zumindest einem elektronischen Gerät zu empfangen, das über das Netzwerk verbunden ist und eine Bioinformationsmessvorrichtung umfasst;
- ein Authentifizierungsmodul, das eingerichtet ist, um auf der Basis der empfangenen Benutzerinformationen eine Benutzerauthentifizierung durchzuführen; und
- ein Datenverarbeitungsmodul, das eingerichtet ist, um auf der Basis der empfangenen Benutzerinformationen und des empfangenen Bioinformationsmesssignals Blutdruckinformationen zu schätzen und die geschätzten Blutdruckinformationen über das Netzwerk an zumindest ein elektronisches Gerät zu übertragen;
- wobei die Bioinformationsmessvorrichtung ein Kamerasensor ist, und
- das Bioinformationsmesssignal ein Bildsignal ist, das einem vom Kamerasensor aufgenommenen Finger oder Gesicht entspricht;
- wobei das Datenverarbeitungsmodul ferner eingerichtet ist, um
- ein erstes RGB-Bild des Bildsignals in ein HSV-Bild umzuwandeln,
- einen V-Kanal-Wert des HSV-Bildes auf einen voreingestellten Wert zu ändern,
- das HSV-Bild, das den voreingestellten Wert als V-Kanal-Wert aufweist, in ein zweites RGB-Bild umzuwandeln,
- ein G-Kanal-Bild aus dem zweiten RGB-Bild zu extrahieren, und
- die Blutdruckinformationen unter Verwendung des G-Kanal-Bildes und eines zuvor maschinell gelernten Blutdruck-Schätzmodells zu schätzen.

8. System zur Bereitstellung medizinischer Informationen auf Basis einer offenen API nach Anspruch 7, wobei zumindest ein elektronisches Gerät eine Smartwatch, ein Smartphone, ein medizinisches Gerät oder eine IoT-Vorrichtung ist.

9. System zur Bereitstellung medizinischer Informationen auf Basis einer offenen API nach Anspruch 8, wobei zumindest ein elektronisches Gerät ein offenes API-Client-Modul umfasst, um das offene API-Protokoll aufzurufen.

10. System zur Bereitstellung medizinischer Informationen auf Basis einer offenen API nach einem der Ansprüche 7 bis 9, wobei das Blutdruck-Schätzmodell aus dem Bild des Grünkanals generiert wird.

## Revendications

1. Procédé de fourniture d'informations médicales basé sur une interface de programmation d'applications (API) ouverte, comprenant :
la réception d'informations d'utilisateur et d'un signal de mesure d'informations biologiques par le biais d'un réseau selon un appel du protocole d'API ouverte dans au moins un appareil électronique qui est connecté par le biais du réseau et inclut un dispositif de mesure d'informations biologiques ;
la réalisation d'une authentification d'utilisateur, sur la base des informations d'utilisateur reçues, et l'estimation d'informations de tension artérielle, sur la base des informations d'utilisateur et du signal de mesure d'informations biologiques ; et
l'émission des informations de tension artérielle estimées vers l'au moins un appareil électronique par le biais du réseau, dans lequel le dispositif de mesure d'informations biologiques est un capteur d'appareil de prise de vues, et le signal de mesure d'informations biologiques est un signal d'image correspondant à un doigt ou à un visage photographié par le capteur d'appareil de prise de vues,
dans lequel l'estimation des informations de tension artérielle comprend :
la transformation d'une première image RGB du signal d'image en une image HSV,
la modification d'une valeur de canal V de l'image HSV en une valeur prédéfinie,
la transformation de l'image HSV ayant la valeur prédéfinie comme étant sa valeur de canal V en une seconde image RGB,
l'extraction d'une image de canal G à partir de la seconde image RGB, et
l'estimation des informations de tension artérielle en utilisant l'image de canal G et un modèle d'estimation de tension artérielle ayant préalablement subi un apprentissage automatique.

2. Procédé de fourniture d'informations médicales basé sur une API ouverte selon la revendication 1, dans lequel l'au moins un appareil électronique est l'un parmi une montre intelligente, un téléphone intelligent, un appareil médical et un dispositif de l'internet des objets, IoT.

3. Procédé de fourniture d'informations médicales basé sur une API ouverte selon la revendication 2, dans lequel l'au moins un appareil électronique inclut un module client d'API ouverte pour appeler le protocole d'API ouverte.

4. Procédé de fourniture d'informations médicales basé sur une API ouverte selon la revendication 1, dans lequel les informations d'utilisateur comprennent une taille, un sexe, un âge et un poids.

5. Procédé de fourniture d'informations médicales basé sur une API ouverte selon l'une quelconque des revendications précédentes, dans lequel le modèle d'estimation de tension artérielle est généré à partir de l'image de canal vert.

6. Support d'enregistrement non transitoire lisible par ordinateur ayant, enregistré sur celui-ci, un programme pour l'exécution du procédé de fourniture d'informations médicales basé sur une API ouverte selon l'une quelconque des revendications 1 à 5.

7. Système de fourniture d'informations médicales basé sur une interface de programmation d'applications (API) ouverte, comprenant :
un module d'API ouverte configuré pour recevoir des informations d'utilisateur et un signal de mesure d'informations biologiques par le biais d'un réseau selon un appel du protocole d'API ouverte dans au moins un appareil électronique qui est connecté par le biais du réseau et inclut un dispositif de mesure d'informations biologiques ;
un module d'authentification configuré pour réaliser une authentification d'utilisateur, sur la base des informations d'utilisateur reçues ; et
un module de traitement de données configuré pour estimer des informations de tension artérielle, sur la base des informations d'utilisateur reçues et du signal de mesure d'informations biologiques reçu, et émettre les informations de tension artérielle estimées vers l'au moins un appareil électronique par le biais du réseau,
dans lequel le dispositif de mesure d'informations biologiques est un capteur d'appareil de prise de vues, et le signal de mesure d'informations biologiques est un signal d'image correspondant à un doigt ou à un visage photographié par le capteur d'appareil de prise de vues.
dans lequel le module de traitement de données est en outre configuré pour transformer une première image RGB du signal d'image en une image HSV, modifier une valeur de canal V de l'image HSV en une valeur prédéfinie,
transformer l'image HSV ayant la valeur prédéfinie comme étant sa valeur de canal V en une seconde image RGB,
extraire une image de canal G à partir de la seconde image RGB, et
estimer les informations de tension artérielle en utilisant l'image de canal G et un modèle d'estimation de tension artérielle ayant préalablement subi un apprentissage automatique.

8. Système de fourniture d'informations médicales basé sur une API ouverte selon la revendication 7, dans lequel l'au moins un appareil électronique est l'un parmi une montre intelligente, un téléphone intelligent, un appareil médical et un dispositif de l'internet des objets, IoT.

9. Système de fourniture d'informations médicales basé sur une API ouverte selon la revendication 8, dans lequel l'au moins un appareil électronique inclut un module client d'API ouverte pour appeler le protocole d'API ouverte.

10. Système de fourniture d'informations médicales basé sur une API ouverte selon l'une quelconque des revendications 7 à 9, dans lequel le modèle d'estimation de tension artérielle est généré à partir de l'image de canal vert.
